# EUROPÄISCHE PATENTSCHRIFT

(11) **EP 0 813 056 B1**
(45) Veröffentlichungstag und Bekanntmachung des Hinweises auf die Patenterteilung: **24.12.2008**
(21) Anmeldenummer: 97106853.1
(22) Anmeldetag: 25.04.1997
(51) Int. Cl.: G01N 27/411

(54) **Vorrichtung zur Durchführung von elektrochemischen Messungen in Glas- oder Salzschmelzen**
Device for carrying out electrochemical measurements in glass or salt melts
Dispositif de réalisation de mesures électrochimiques dans des verres ou des sels en fusion

(30) Priorität: 14.06.1996 DE 19623687
(43) Veröffentlichungstag der Anmeldung: 17.12.1997
(73) Patentinhaber: Heraeus Electro-Nite International N.V., 3530 Houthalen (BE)
(72) Erfinder: Plessers, Jacques Josef, Dr., 3530 Houthalen (BE); Straetemans, Marc, 3941 Eksel (BE)
(74) Vertreter: Kühn, Hans-Christian

(56) Entgegenhaltungen:
- DE-A- 3 303 851
- FR-A- 2 122 758
- GB-A- 2 057 695
- US-A- 3 625 026
- US-A- 4 313 799
- J. ELECTROCHEM. SOC., Bd. 119, Nr. 2, Februar 1972, Seiten 198-208, XP002064944
- MÜLLER-SIMON H.; MERGLER K.W.: 'Sensor for oxygen activity measurements in glass melts' GLASTECHNISCHE BERICHTE Bd. 64, Nr. 2, 1991, FRANKFURT,

## Beschreibung

Die Erfindung betrifft eine Vorrichtung zur Durchführung von elektrochemischen Messungen in Glas- oder Salzschmelzen mit wenigstens einer Meßelektrode und einer Referenzelektrodenanordnung.

Derartige Vorrichtungen sind vielfach, beispielsweise aus GB 2 057 695 A bekannt. Hier erfolgt eine Messung des Sauerstoffpartialdruckes mittels einer elektrochemischen Meßzelle, auch Referenzelektrodenanordnung genannt, die über eine übliche Anzeige- und/oder Auswerteeinrichtung (Meßsystem) mit einer Gegenelektrode (auch Meßelektrode) verbunden ist. Als Meßelektrode wird ein Platindraht verwendet, der durch einen Aluminiumoxid-Körper hindurchgeführt ist. An der Spitze des Aluminiumoxid-Körpers ist der Platindraht freiliegend, so daß er in Kontakt mit der Schmelze treten kann, sobald die Gegenelektrode in diese eintaucht. Der Aluminiumoxid-Körper ist in einem Aluminiumoxidrohr gehaltert. In der Praxis hat es sich gezeigt, daß es nicht möglich ist, eine gasdichte Durchführung zwischen der Platinelektrode und dem Aluminiumoxid-Körper zu schaffen. Dadurch dringt Sauerstoff aus der Atmosphäre oberhalb der Schmelze bis zu dem mit der Schmelze in Kontakt stehenden Teil der Meßelektrode, so daß die dort gemessenen Werte nicht den tatsächlichen Verhältnissen innerhalb der Schmelze entsprechen und die Messung dadurch fehlerbehaftet ist.

Ähnliche Meßanordnungen sind beispielsweise auch aus DE 38 11 915 A1 bekannt. Auch hier ist die Meßelektrode aus Platin gebildet.

Beispielsweise aus "Glastechnische Berichte" 68 (1995) No. 9, S. 273 ff. ist es bekannt, durch voltametrische Analyse mit drei Elektroden Eisen, Schwefel oder Chrom in Glasschmelzen zu bestimmen. Auch hier treten die genannten Probleme auf. So muß zum Beispiel die Größe der Elektrodenoberfläche im Glas genau bekannt sein.

T.H. Etsell bestimmt voltammetrisch Sauerstoff in Metallschmelzen (Etsell, T.H., J. Electrochem. Soc.: Electrochemical Science and Technology; Feb. 1972; p. 198-208).

Aufgabe der vorliegenden Erfindung ist es, ausgehend von den aus dem Stand der Technik bekannten Vorrichtungen die Meßgenauigkeit von beispielsweise Sauerstoffpartialdruckmessungen in Glas- oder Salzschmelzen zu verbessern.

Erfindungsgemäß wird die Aufgabe dadurch gelöst, daß die zum Eintauchen in die Schmelze bestimmte Spitze der Meßelektrode aus einem Edelmetall gebildet ist und in einem Ende eines Quarzglasrohr gasdicht eingeschmolzen ist, wobei die Meßelektrode durch das Quarzglasrohr hindurchgeführt ist (aus der Schmelze heraus zur Auswerteeinrichtung). Eine gasdichte Halterung bedeutet, daß kein Sauerstoff durch das Rohr von außen bis zur zu messenden Schmelze in einer solchen Menge dringt, die die Messung beeinflußt. Zweckmäßigerweise ist das Edelmetall ein Metall der Gruppe Iridium, Platin, Palladium oder Rhodium oder eine Legierung mindestens eines dieser Metalle mit mindestens einem weiteren Edelmetall (eventuell auch aus dieser Gruppe). Es ist denkbar, die Vorrichtung überwiegend für Kurzzeitmessungen einzusetzen.

Zweckmäßig ist es, daß die Verbindung zwischen Meßelektrode und Quarzglasrohr an dem zum Eintauchen in die Schmelze bestimmten Ende des Quarzglasrohres gasdicht eingeschmolzen ist. Nach hinten, aus der Schmelze heraus, kann das Quarzglasrohr offen sein. Zweckmäßig ist es, daß die aus Edelmetall gebildete Spitze der Meßelektrode innerhalb des Quarzglasrohres mit einem Meßdraht verbunden ist, der vorzugsweise aus Molybdän, Wolfram oder einer Chrom-Nickel-Legierungen (z. B. Cronix) gebildet ist. Dadurch kann die Länge des als Elektrode verwendeten Edelmetall-Drahtes kurzgehalten werden, um Edelmetall einzusparen.

Im Falle der Ausbildung des Meßdrahtes aus Molybdän oder Wolfram ist es möglich, zwischen der aus Edelmetall gebildeten Spitze der Meßelektrode und dem Meßdraht einen Metallstreifen aus Molybdän anzuordnen. Die Verbindung zwischen Edelmetall und Meßdraht kann in dem Quarzglasrohr eingeschmolzen sein. Insbesondere ein eingeschmolzener Metallstreifen aus Molybdän sichert eine nahezu absolute Gasdichtheit.

Ein Chrom-Nickel-Draht kann nicht ohne weiteres in das Quarzglasrohr eingeschmolzen werden, da die Gefahr besteht, daß es bei der notwendigen Temperatur schmilzt. Die Verbindungsstelle ist daher vorzugsweise hinter der Einschmelzstelle in dem Quarzglasrohr anzuordnen.

Vorteilhaft für eine hohe Meßgenauigkeit ist es, daß eine Referenzelektrode in einem einseitig geschlossenen Festelektrolytröhrchen angeordnet ist, das mit seinem dem geschlossenen Ende abgewandten Ende in einem Keramikrohr gehaltert ist, durch das die Referenzelektrode hindurchgeführt ist und daß das Ende der Referenzelektrode in dem Festelektrolytröhrchen von einem Referenzmaterial umgeben ist, daß aus einer Metall-Metalloxid-, vorzugsweise einer Nickel-Nickel-Oxid-Pulvermischung gebildet ist; die Referenzelektrode selbst ist zweckmäßigerweise aus einer Chrom-Nickel-Legierung gebildet.

Zweckmäßig ist es weiterhin, daß das Quarzglasrohr und das Keramikrohr mit Korund gefüllt sind. Desweiteren ist es vorteilhaft, daß das Quarzglasrohr und das Keramikrohr in einem gemeinsamen Trägerrohr gehaltert sind, das vorzugsweise aus Keramik gebildet ist und das an seinem dem Eintauchende abgewandten Ende ein Verbindungsstück üblicher Art aufweist zur mechanischen Ankopplung und zur Verbindung von Meßelektrode und Referenzelektrode mit einem Meßsystem. Das Trägerrohr kann aus Aluminiumoxid gebildet und mit Kugelkorund gefüllt sein.

Nachfolgend wird ein Ausführungsbeispiel der Erfindung anhand einer Zeichnung näher erläutert.

In der Zeichnung zeigt
- Figur 1: eine Darstellung der erfindungsgemäßen Vorrichtung,
- Figur 2: einen Längsschnitt durch die erfindungsgemäße Vorrichtung,
- Figur 3: einen Längsschnitt durch die in das Quarzglasrohr eingeschmolzene Meßelektrode und
- Figur 4: einen Schnitt durch eine Vorrichtung mit drei Elektroden.

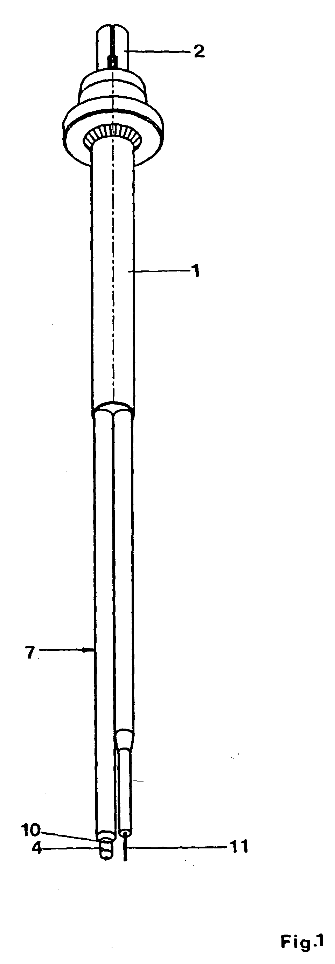

Die in Figur 1 dargestellte Vorrichtung weist ein Trägerrohr 1 auf, das aus Aluminiumoxid gebildet ist. An seinem dem Eintauchende abgewandten Ende ist ein Verbindungsstück 2 an dem Trägerrohr 1 angeordnet, das in einen in der Figur nicht dargestellten Halter, beispielsweise eine metallene Lanze eingesteckt wird. Die durch das Trägerrohr 1 hindurchgeführten Drähte, der Meßdraht 3 und die Referenzelektrode 4 werden über das Verbindungsstück 2 mit einem Meßsystem, das heißt mit einer üblichen Anzeige- und/oder Auswerteeinheit verbunden. Innerhalb des Trägerrohres 1 sind der Meßdraht 3 und die Referenzelektrode 4 durch Quarzglasröhrchen 5 geführt und in Kugelkorund 6 eingebettet.

An dem dem Eintauchende der Vorrichtung zugewandten Ende des Trägerrohres 1 sind die Referenzelektrodenanordnung 7 und das Quarzglasrohr 8 angeordnet. Die Referenzelektrodenanordnung 7 weist ein Keramikrohr 9 aus Aluminiumoxid auf, durch das die Referenzelektrode 4 bis in das Festelektrolytröhrchen 10 hineingeführt ist. Das Festelektrolytröhrchen 10 aus Zirkonoxid weist in seinem Inneren als Referenzmaterial eine Nickel-Nickeloxid-Pulvermischung auf, in der die Referenzelektrode 4, die aus einer Chrom-Nickel-Legierung (Cronix) gebildet ist, gehaltert ist. Durch das Quarzglasrohr 8 hindurch ist die Meßelektrode mit dem Meßdraht 3 geführt, die Spitze 11 der Meßelektrode ist aus Iridium-Draht gebildet. Sie kann jedoch auch aus Platin, Palladium, Rhodium oder einer Legierung, die überwiegend Iridium oder andere Metalle der vorgenannten Gruppe und darüber hinaus andere Edelmetalle enthält, gebildet sein. Die Spitze 11 aus Iridium ragt bis in das Quarzglasrohr 8 hinein. Die Spitze 11 ist in dem Ende 12 des Quarzglasrohres 8 gasdicht eingeschmolzen auf einer Strecke von etwa 2 cm. Danach wechselt das Material der Meßelektrode. Um den relativ teuren Iridium-Draht einzusparen, ist der Rest der Meßelektrode ein Meßdraht 3 aus Cronix (einer Chrom-Nickel-Legierung). Statt Cronix kann beispielsweise auch Molybdän oder Wolfram als Meßdraht 3 verwendet werden.

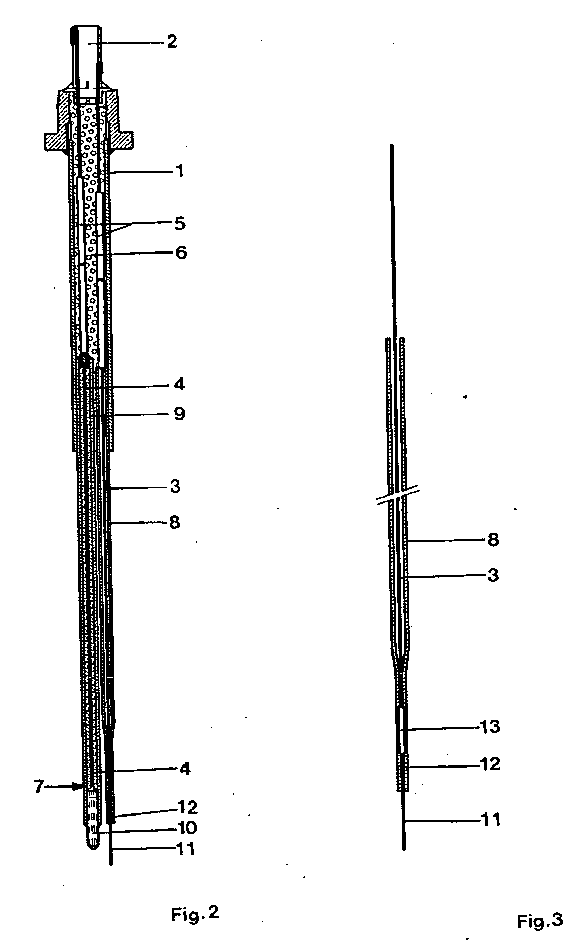

Eine weitere Möglichkeit, die Meßelektrode auszubilden, ist in Figur 3 dargestellt. Hier ist die Spitze 11 aus Iridium innerhalb des Quarzglasrohres 8 mit einem Molybdän-Streifen 13 verbunden, der an seinem anderen Ende mit dem Meßdraht 3 verbunden ist. Der Meßdraht 3 kann in diesem Fall beispielsweise aus Molybdän oder Wolfram gebildet sein. Der Molybdän-streifen 13 ist in dem gezeigten Beispiel vollständig in das Ende 12 des Quarzglasrohres 8 eingeschmolzen. Dadurch kann eine perfekte Gasdichtheit erzielt werden.

In den Figuren nicht dargestellt ist die Möglichkeit, den Molybdän-Streifen 13 aus dem zugeschmolzenen Ende 12 des Quarzglasrohres 8 herauszuführen und erst in dem offenen Rohr mit dem Meßdraht 3 zu verbinden. In einem solchen Fall wäre auch Cronix als Meßdraht 3 möglich.

Quarzglasrohr 8 und Keramikrohr 9 sind mit einer Korundfüllung versehen, die die Lage der Drähte innerhalb der Rohre stabilisiert.

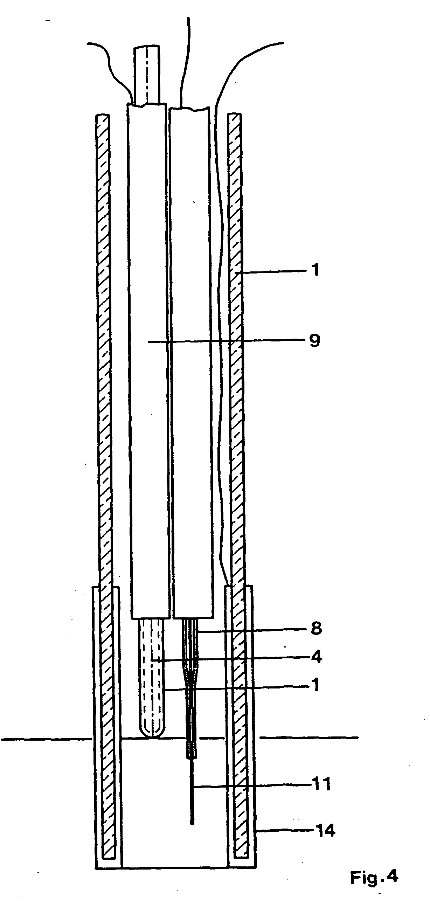

Die in Figur 4 dargestellte Vorrichtung ist zur voltametrischen Messung z. B. des Eisen-, Schwefel- oder Chromgehaltes in einer Glasschmelze geeignet. Das Verfahren hierzu ist beispielsweise in "Glastechnische Berichte" 68 (1995) No. 9, S. 273 ff. beschrieben. In dem Trägerrohr 1 aus Alumiumoxid sind eine Meßelektrode und eine Referenzelektrode 4 angeordnet. Die Spitze 11 der Meßelektrode ist aus Iridium gebildet und in ein Quarzglasrohr 8 eingeschmolzen. Die Referenzelektrode 4 aus Platin ist in einem Keramikrohr 9 angeordnet, und an dem Eintauchende des Trägerrohres 1 ist eine Gegenelektrode 14 aus Platin angeordnet.

Die Messung mit der beschriebenen Vorrichtung ermöglicht sehr zuverlässige Ergebnisse, in erster Linie im Kurzzeitbetrieb. Da die Vorrichtung sehr kostengünstig hergestellt werden kann, ist die Ausbildung als Einwegsonde möglich.

## Patentansprüche

1. Vorrichtung zur Durchführung von elektrochemischen Messungen in Glas- oder Salzschmelzen mit wenigstens einer Meßelektrode und einer Referenzelektrodenanordnung, **dadurch gekennzeichnet, daß** die zum Eintauchen in die Schmelze bestimmte Spitze (11) der Meßelektrode aus einem Edelmetall gebildet ist und in einem Quarzglasrohr (8) gasdicht eingeschmolzen ist, wobei die Meßelektrode durch das Quarzglasrohr (8) hindurchgeführt ist.

2. Vorrichtung nach Anspruch 1, **dadurch gekennzeichnet, daß** das Edelmetall ein Metall der Gruppe Iridium, Platin, Palladium oder Rhodium oder eine Legierung mindestens eines dieser Metalle mit mindestens einem weiteren Edelmetall ist.

3. Vorrichtung nach Anspruch 1 oder 2, **dadurch gekennzeichnet, daß** eine Verbindung zwischen Meßelektrode und Quarzglasrohr (8) an dem zum Eintauchen in die Schmelze bestimmten Ende (12) des Quarzglasrohres (8) eingeschmolzen ist.

4. Vorrichtung nach einem der Ansprüche 1 bis 3, **dadurch gekennzeichnet, daß** die aus Edelmetall gebildete Spitze (11) der Meßelektrode innerhalb des Quarzglasrohres (8) mit einem Meßdraht (3) verbunden ist.

5. Vorrichtung nach Anspruch 4, **dadurch gekennzeichnet, daß** zwischen der aus Edelmetall gebildeten Spitze (11) der Meßelektrode und dem Meßdraht (3) ein Metallstreifen (13) aus Molybdän angeordnet ist.

6. Vorrichtung nach Anspruch 4, **dadurch gekennzeichnet, daß** der Meßdraht (3) aus Molybdän oder Wolfram gebildet ist.

7. Vorrichtung nach einem der Ansprüche 4 bis 6, **dadurch gekennzeichnet, daß** die Verbindung zwischen Edelmetall und dem Meßdraht (3) in dem Quarzglasrohr (8) eingeschmolzen ist.

8. Vorrichtung nach Anspruch 4, **dadurch gekennzeichnet, daß** der Meßdraht (3) aus einer Chrom-Nickel-Legierung gebildet ist.

9. Vorrichtung nach einem der Ansprüche 1 bis 8, **dadurch gekennzeichnet, daß** eine Referenzelektrode (4) in einem einseitig geschlossenen Festelektrolytröhrchen angeordnet ist, das mit seinem dem geschlossenen Ende gegenüberliegenden Ende in einem Keramikrohr (9) gehaltert ist, durch das die Referenzelektrode (4) hindurchgeführt ist und daß das Ende der Referenzelektrode (4) in dem Festelektrolytröhrchen (10) von einem Referenzmaterial umgeben ist, das aus einer Metall-Metalloxid-, vorzugsweise einer Nickel-Nickeloxid-Pulvermischung gebildet ist.

10. Vorrichtung nach einem der Ansprüche 1 bis 9, **dadurch gekennzeichnet, daß** die Referenzelektrode (4) aus einer Chrom-Nickel-Legierung gebildet ist.

11. Vorrichtung nach Anspruch 9 oder 10, **dadurch gekennzeichnet, daß** das Quarzglasrohr (8) und das Keramikrohr (9) mit Korund gefüllt sind.

12. Vorrichtung nach einem der Ansprüche 9 bis 11, **dadurch gekennzeichnet, daß** das Quarzglasrohr (8) und und das Keramikrohr (9) in einem gemeinsamen Trägerrohr (1) gehaltert sind, das vorzugsweise aus Keramik gebildet ist und das an seinem dem Eintauchende abgewandten Ende ein Verbindungsstück (2) aufweist zur mechanischen Ankopplung und zur Verbindung von Meßelektrode und Referenzelektrode (4) mit einem Meßsystem.

13. Vorrichtung nach Anspruch 12, **dadurch gekennzeichnet, daß** das Trägerrohr (1) aus Aluminiumoxid gebildet ist und daß es mit Kugelkorund gefüllt ist.

## Claims

1. An apparatus for taking electrochemical measurements in glass or salt melts, comprising at least one measuring electrode and one reference electrode arrangement, **characterised in that** the tip (11) of the measuring electrode that is intended to be immersed in the melt is formed from a noble metal and is fused in a quartz glass tube (8) in a gastight manner, wherein the measuring electrode is passed through the quartz glass tube (8).

2. The apparatus according to Claim 1, **characterised in that** the noble metal is a metal of the group comprising iridium, platinum, palladium or rhodium or of an alloy of at least one of these metals and at least one further noble metal.

3. The apparatus according to Claim 1 or 2, **characterised in that** a connection between the measuring electrode and the quartz glass tube (8) is fused in at the end (12) of the quartz glass tube (8) that is intended to be immersed in the melt.

4. The apparatus according to anyone of Claims 1 to 3, **characterised in that** the tip (11) of the measuring electrode that is formed from a noble metal is connected to a measuring wire (3) inside the quartz glass tube (8).

5. The apparatus according to Claim 4, **characterised in that** a metal strip (13) made of molybdenum is arranged between the tip (11) of the measuring electrode that is formed from a noble metal and the measuring wire (3).

6. The apparatus according to Claim 4, **characterised in that** the measuring wire (3) is formed from molybdenum or tungsten.

7. The apparatus according to anyone of Claims 4 to 6, **characterised in that** the connection between the noble metal and the measuring wire (3) is fused in the quartz glass tube (8).

8. The apparatus according to Claim 4, **characterised in that** the measuring wire (3) is formed from a chrome nickel alloy.

9. The apparatus according to anyone of Claims 1 to 8, **characterised in that** a reference electrode (4) is arranged in a solid electrolyte tube which is closed on one side and which, with its end opposite to the closed end, is held in a ceramic tube (9) through which the reference electrode (4) is passed, and that the end of the reference electrode (4) in the solid electrolyte tube (10) is surrounded by a reference material which is formed from a powder mixture comprising a metal and metal oxide, preferrably from a powder mixture comprising nickel and nickel oxide.

10. The apparatus according to anyone of Claims 1 to 9, **characterised in that** the reference electrode (4) is formed from a chrome nickel alloy.

11. The apparatus according to Claim 9 or 10, **characterised in that** the quartz glass tube (8) and the ceramic tube (9) are filled with corundum.

12. The apparatus according to anyone of Claims 9 to 11, **characterised in that** the quartz glass tube (8) and the ceramic tube (9) are held in a common carrier tube (1) which is, preferrably, formed from ceramic and, at its end facing away from the immersion end, comprises a connecting piece (2) for mechanical coupling and for connecting the measuring electrode and the reference electrode (4) to a measuring system.

13. The apparatus according to Claim 12, **characterised in that** the carrier tube (1) is formed from an aluminium oxide and that it is filled with spherical corundum.

## Revendications

1. Appareil pour réaliser des mesures électrochimiques dans des verres ou des sels en fusion, avec au moins une électrode de mesure et un agencement à électrode de référence, **caractérisé en ce que** la pointe (11) de l'électrode de mesure, destinée à plonger dans le matériau en fusion, est formée en métal noble et est intégrée par fusion de façon étanche au gaz dans un tube en verre de quartz (8), ladite électrode de mesure étant passée à travers le tube en verre de quartz (8).

2. Appareil selon la revendication 1, **caractérisé en ce que** le métal noble est un métal du groupe comprenant iridium, platine, palladium ou rhodium ou un alliage d'au moins un de ces métaux avec au moins un autre métal noble.

3. Appareil selon la revendication 1 ou 2, **caractérisé en ce qu'**une jonction entre l'électrode de mesure et le tube en verre de quartz (8) est intégrée par fusion à l'extrémité (12) du tube en verre de quartz (8) destinée à plonger dans le matériau en fusion.

4. Appareil selon l'une des revendications 1 à 3, **caractérisé en ce que** la pointe (11) de l'électrode de mesure, réalisée en métal noble, est reliée à l'intérieur du tube en verre de quartz (8) à un fil de mesure (3).

5. Appareil selon la revendication 4, **caractérisé en ce qu'**un ruban métallique (13) en molybdène est agencé entre la pointe (11) de l'électrode de mesure, réalisée en métal noble, et le fil de mesure (3).

6. Appareil selon la revendication 4, **caractérisé en ce que** le fil de mesure (3) est réalisé en molybdène ou en tungstène.

7. Appareil selon l'une des revendications 4 à 6, **caractérisé en ce que** la jonction entre le métal noble et le fil de mesure (3) est intégrée par fusion dans le tube en verre de quartz (8).

8. Appareil selon la revendication 4, **caractérisé en ce que** le fil de mesure (3) est réalisé en un alliage chrome-nickel.

9. Appareil selon l'une des revendications 1 à 8, **caractérisé en ce qu'**une électrode de référence (4) est agencée dans un petit tube à électrolyte solide fermé sur un côté, lequel est maintenu par son extrémité opposée à l'extrémité fermée dans un tube en céramique (9) à travers lequel est passée l'électrode de référence (4), et **en ce que** l'extrémité de l'électrode de référence (4) dans le petit tube à électrolyte solide (10) est entourée par un matériau de référence qui est formé par un mélange de poudre métal/oxyde métallique, de préférence un mélange de poudre nickel/oxyde de nickel.

10. Appareil selon l'une des revendications 1 à 9, **caractérisé en ce que** l'électrode de référence (4) est réalisée en un alliage chrome-nickel.

11. Appareil selon la revendication 9 ou 10, **caractérisé en ce que** le tube en verre de quartz (8) et le tube en céramique (9) sont remplis de corindon.

12. Appareil selon l'une des revendications 9 à 11, **caractérisé en ce que** le tube en verre de quartz (8) et le tube en céramique (9) sont maintenus dans un tube porteur commun (1), lequel est de préférence réalisé en céramique et comporte à son extrémité détournée de l'extrémité plongeante une pièce de liaison (2) pour le couplage mécanique et pour la connexion de l'électrode de mesure et de l'électrode de référence (4) à un système de mesure.

13. Appareil selon la revendication 12, **caractérisé en ce que** le tube porteur (1) est réalisé en oxyde d'aluminium et **en ce qu'**il est rempli de billes de corindon.
